(19) Europäisches Patentamt
European Patent Office
Office européen des brevets

(11) Numéro de publication: **0 402 232 A1**

(12) **DEMANDE DE BREVET EUROPEEN**

(21) Numéro de dépôt: **90401512.0**

(22) Date de dépôt: **05.06.90**

(51) Int. Cl.[5]: **C07D 471/04, A61K 31/435, //(C07D471/04,221:00,209:00)**

(30) Priorité: **06.06.89 FR 8907450**
**25.04.90 FR 9005059**

(43) Date de publication de la demande:
**12.12.90 Bulletin 90/50**

(84) Etats contractants désignés:
**AT BE CH DE DK ES FR GB GR IT LI LU NL SE**

(71) Demandeur: **RHONE-POULENC SANTE**
**20, avenue Raymond Aron**
**F-92165 Antony Cédex(FR)**

(72) Inventeur: **Bisagni, Emile**
**16, Rue Bossuet**
**F-91400 Orsay(FR)**
Inventeur: **Nguyen, Chi-Hung**
**6, Place des Italiens**
**F-91300 Massy(FR)**

(74) Mandataire: **Savina, Jacques et al**
**RHONE-POULENC SANTE, Service Brevets,**
**20 Avenue Raymond Aron**
**F-92165 Antony Cédex(FR)**

(54) **Nouveaux dérivés de pyridobenzoindole, leur préparation et les compositions qui les contiennent.**

(57) Nouveaux dérivés de pyridobenzoindole de formule générale (I), dans laquelle R est H ou alcoyle (1 ou 2 C), alk est alcoylène (2 à 4 C) droit ou ramifié, $R_1$ représente un atome d'hydrogène ou un radical alcoyle (1 ou 2 C), $R_2$ représente un radical hydroxy ou méthoxy et $R_3$ est méthyle ou éthyle et leurs sels d'addition avec les acides, utiles comme agents anti-tumoraux.

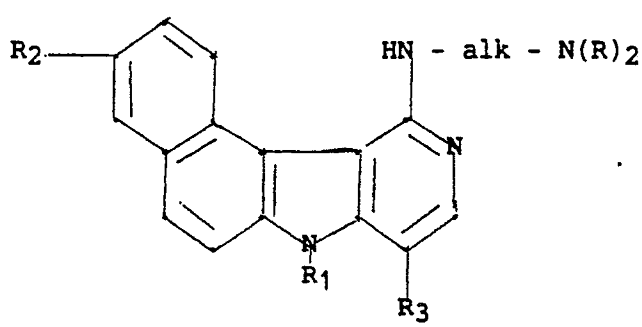

(I)

EP 0 402 232 A1

# NOUVEAUX DERIVES DE PYRIDOBENZOINDOLE, LEUR PREPARATION ET LES COMPOSITIONS QUI LES CONTIENNENT

La présente invention concerne de nouveaux dérivés de pyridobenzoindole de formule générale :

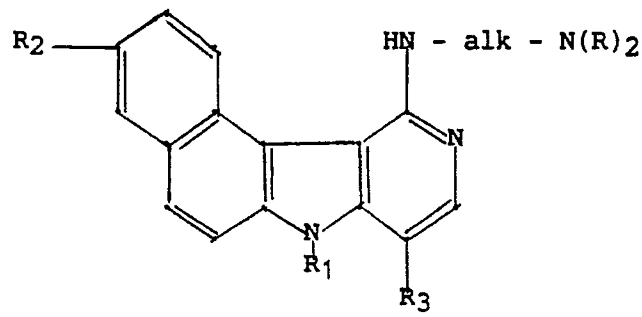

(I)

le cas échéant leur hydrates, leurs sels d'addition avec les acides, leur préparation et les compositions pharmaceutiques qui les contiennent.

Dans la demande de brevet européen 239 476 ont été décrits des dérivés de γ-carboline de formule générale :

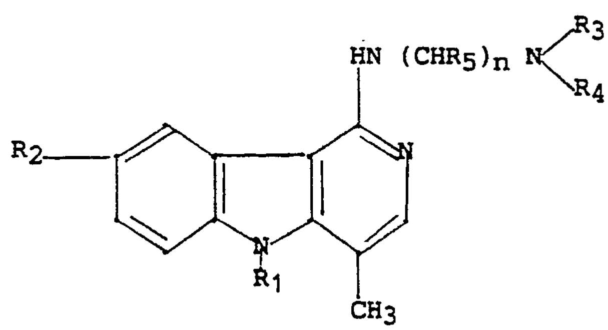

dans laquelle $R_1$ et $R_5$ sont entre autres des atomes d'hydrogène, $R_2$ peut représenter un atome d'hydrogène, un radical hydroxy ou alcoyloxy, $R_3$ et $R_4$ sont notamment des radicaux alcoyle et n égale 2 à 4, qui présentent une activité anti-tumorale.

Par ailleurs, d'autres dérivés du pyridoindole ont été décrits dans la littérature par C. DUCROCQ et coll., J.Het Chem., 12 (5), 963 (1975) ou par Ch.S. LEE et coll., Heterocycles, 16 (7), 1081 (1981) mais aucune activité thérapeutique n'est mentionnée.

Les nouveaux dérivés du pyridobenzoindole de formule générale (I) dans laquelle:
R représente un atome d'hydrogène ou un radical alcoyle contenant 1 ou 2 atomes de carbone,
alk représente un radical alcoylène droit ou ramifié contenant 2 à 4 atomes de carbone, $R_1$ représente un atome d'hydrogène ou un radical alcoyle contenant 1 ou 2 atomes de carbone,
$R_2$ représente un radical hydroxy ou méthoxy, et
$R_3$ représente un radical alcoyle contenant 1 ou 2 atomes de carbone, présentent des propriétés anti-tumorales particulièrement intéressantes.

Selon l'invention, les produits de formule générale (I) peuvent être obtenus à partir du dérivé chloré de formule générale :

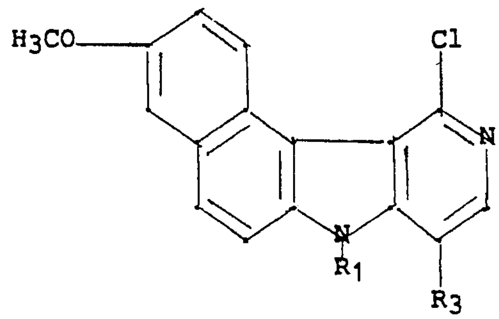

(II)

dans laquelle $R_1$, est défini comme précédemment par action d'une amine de formule générale :
$H_2N$ - alk - $N(R)_2$ (III)
dans laquelle alk et R sont définis comme précédemment, suivie le cas échéant de la transformation du produit méthoxylé ainsi obtenu, de formule générale

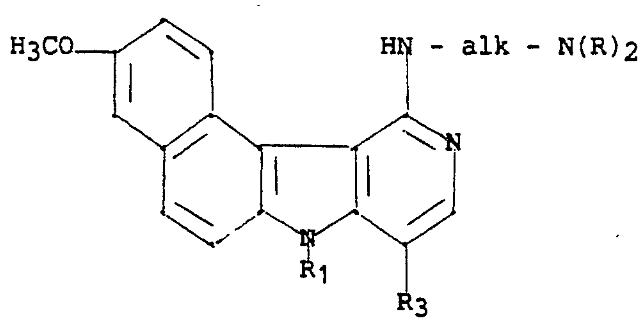

(Ia)

dans laquelle R, $R_1$, $R_3$ et alk sont définis comme ci-dessus, un dérivé hydroxy-9 pyrido[4,3-b]benzo[e]-indole.

La réaction de l'amine de formule générale (III) sur le dérivé chloré de formule générale (II) s'effectue en présence d'un excés de l'amine, de préférence sous azote, éventuellement dans un solvant organique inerte, ou sans solvant, à une température comprise entre la température de reflux du mélange réactionnel, et 250° C (autoclave).

La déméthylation s'effectue par toute méthode connue qui n'altère pas le reste de la molécule.

Le dérivé chloré de formule générale (II) pour lequel $R_1$, est un atome d'hydrogène peut être préparé à partir de la pyridone correspondante, de formule générale:

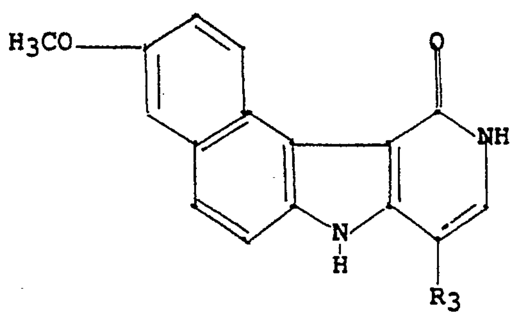

(IV)

dans laquelle $R_3$ est défini comme précédemment, par action d'un agent de chloration.

On opère généralement au moyen d'un agent de chloration choisi parmi l'oxychlorure de phosphore ou les dérivés halogénés du phosphore en présence d'une base tertiaire (diéthylaniline, diméthylaniline par exemple) dans un solvant organique tel qu'un un nitrile (acétonitrile par exemple), à une température comprise entre 75 et 90° C (température de reflux du mélange réactionnel).

De préférence, la réaction s'effectue sous azote.

Le produit de formule générale (II) pour lequel $R_1$ et un radical alcoyle peut-être obtenu par alkylation du produit pour lequel $R_1$ est un atome d'hydrogène par exemple par action du dérivé halogéné

convenable, en présence de carbonate de potassium.

Le produit de formule (IV) peut être préparé à partir de l'hydrazone de formule générale:

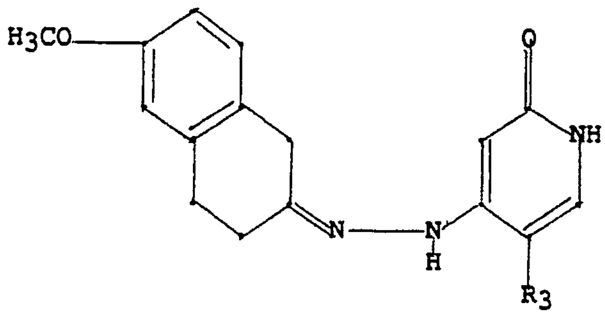

(V)

dans laquelle $R_3$ est défini comme précédemment par réaction de Fisher thermique, par analogie avec la méthode décrite par C.H. NGUYEN et E. BISAGNI, Tetrahedron, 42 (8), 2203 (1986).

L'hydrazone de formule générale (V) est préparée par condensation d'hydrazino-4 méthyl(ou éthyl)-5-1H-pyridone-2 avec la méthoxy-6 tétralone-2. La réaction s'effectue généralement dans un solvant organique tel qu'un alcool (éthanol par exemple), à la température de reflux du mélange réactionnel.

La méthoxy-6 tétralone peut être obtenue selon la méthode décrite dans J. Am. Chem. Soc., 82, 2573 (1960).

L'hydrazino-4 méthyl-5-1H-pyridone-2 peut être préparée selon la méthode décrite par C.H. NGUYEN et E. BISAGNI, Tetrahedron, 42 (8), 2203 (1986).

Les dérivés de pyridobenzoindole de formule générale (I) peuvent être purifiés par cristallisation ou par chromatographie.

Les nouveaux dérivés de pyridobenzoindole selon l'invention peuvent être transformés en sels d'addition avec les acides, par action d'un acide dans un solvant organique. Le sel précipite, éventuellement après concentration de sa solution, il est séparé par filtration ou décantation.

Comme sels pharmaceutiquement acceptables, on peut citer les sels d'addition avec les acides minéraux tels que chlorhydrates, bromhydrates, sulfates, nitrates, phosphates, ou organiques tels que acétate, propionates, succinates, maléates, fumarates, méthanesulfonates, p.toluènesulfonates, iséthionates ou des dérivés de substitution de ces composés.

Les dérivés de pyridobenzoindole, selon l'invention et leurs sels peuvent exister à l'état d'hydrates, il est entendu que ces formes hydratées entrent aussi dans le cadre de la présente invention.

De plus lorsque le symbole alk est un radical alcoylène ramifié, les dérivés du pyridobenzoindole présentent des formes isomères. Il est entendu que ces formes isomères entrent aussi dans le cadre de l'invention.

Les dérivés de formule générale (I) sont particulièrement intéressants comme agents anti-tumoraux.

Leurs propriétés anti-tumorales ont notamment été mises en évidence à une concentration voisine de 15 µg/disque dans le test de cytotoxicité différentielle, selon la technique décrite par T.H. Corbett et coll., Investigational new drug, 4, 207-220 (1986), et sur les tumeurs greffées de la souris, plus particulièrement sur leucémie P388, les produits étudiés se sont montrés actifs à des doses comprises entre 5 et 20 mg/kg par voie intrapéritonéale.

Leur toxicité chez la souris exprimée par leur dose maximale tolérée est comprise entre 10 et 20 mg/kg par voie intrapéritonéale.

D'un intérêt particulier pour leur activité anti-tumorale sont principalement les dérivés du pyridobenzoindole de formule générale (I), pour lesquels:

R est un atome d'hydrogène ou un radical méthyle, alk est un radical alcoylène droit ou ramifié contenant 2 à 4 atomes de carbone, $R_1$ est un atome d'hydrogène ou un radical méthyle, $R_2$ est un radical hydroxy et $R_3$ est un radical méthyle ou éthyle, ainsi que leurs sels et le cas échéant leurs hydrates.

Et parmi ces produits, plus spécialement intéressants sont les dérivés du pyridobenzoindole pour lesquels:

R est un radical méthyle, alk est un radical alcoylène droit ou ramifié contenant 2 à 4 atomes de carbone, $R_1$ est un atome d'hydrogène ou un radical méthyle, $R_2$ est un radical hydroxy et $R_3$ est un radical méthyle, ainsi que leurs sels et le cas échéant leurs hydrates.

Notamment

- Le [(diméthylamino-3) propyl] amino-1 hydroxy-9 méthyl-4-5H-pyrido[4,3-b] benzol[e]indole, ainsi que ses

sels et ses formes hydratées,

- Le (diméthylamino-3 méthyl-2 propyl)amino-1 hydroxy-9 méthyl-4 5H-pyrido [4,3-b] benzo[e]indole, ainsi que ses sels et ses formes hydratées.

Les exemples suivants donnés à titre non limitatif illustrent la présente invention.

EXEMPLE 1

Le [(diméthylamino-3)propyl]amino-1 méthoxy-9 méthyl-4-5H-pyrido [4,3-b] benzo [e] indole (8,7 g) est placé dans un ballon réactionnel de 500 $cm^3$ muni d'un agitateur et maintenu sous argon, avec 220 $cm^3$ d'acide bromhydrique (d = 1,47, 47 %).

Ce mélange, devenu homogène à l'ébullition, est chauffé à reflux pendant 4 heures et concentré à sec sous pression réduite. Le résidu solide est dissous dans l'eau (800 $cm^3$) alcalinisé par l'ammoniaque à 28 % (85 $cm^3$) ajouté goutte à goutte, pour former un précipité gommeux.

Au mélange résultant, on ajoute 800 $cm^3$ d'acétate d'éthyle. L'ensemble est agité pendant 1 heure et filtré pour éliminer une petite fraction insoluble. La phase organique est décantée et les eaux mères sont extraites deux fois avec 400 $cm^3$ d'acétate d'éthyle. La phase organique est séchée sur sulfate de sodium, filtrée, traitée avec 16 à 20 g de charbon animal pour la décolorer, filtrée et concentrée à sec.

Le résidu solide est repris dans 80 $cm^3$ d'acétone, agité pendant 1 heure et filtré puis lavé 2 fois avec 15 $cm^3$ d'acétone froide. Le traitement dans l'acétone est répété une fois pour donner 6,3 g de poudre cristalline jaune-beige, F = 216-218 °C, correspondant au [(diméthylamino-3)propyl]amino-1 hydroxy-9 méthyl-4-5H-pyrido [4,3-b] benzo [e] indole.

Le [(diméthylamino-3)propyl]amino-1 hydroxy-9 méthyl-4-5H-pyrido [4,3-b] benzo [e] indole (7,5 g) en solution dans la méthyléthylcétone bouillante (650 $cm^3$), est ajouté à une solution d'acide méthanesulfonique (6,4 g, 3 équivalents) dans 300 $cm^3$ de méthyléthylcétone bouillante et le mélange est maintenu à reflux pendant 5 minutes. Il se forme une masse gommeuse insoluble, qui se délite progressivement en une poudre grise, en 18 heures sous agitation. Le solide est filtré, lavé avec la méthyléthylcétone et mis rapidement dans un dessicateur contenant de l'anhydride phosphorique. Après 18 heures, sous pression réduite (15 mm de mercure), on obtient 11,7 g (97 %) du di-méthanesulfonate de [(diméthylamino-3)-propyl]amino-1 hydroxy-9 méthyl-4-5H-pyrido [4,3-b] benzo [e] indole, F = 245-255 °C.

Une solution de [(diméthylamino-3)propyl]amino-1 hydroxy-9 méthyl-4-5H-pyrido [4,3-b] benzo [e] indole (670 mg) dans le méthanol bouillant (150 $cm^3$) est versée dans 100 $cm^3$ de méthanol saturé par l'acide chlorhydrique. La solution est concentrée jusqu'à 30$cm^3$ et additionnée de 60 $cm^3$ d'acétone. Le précipité est filtré après 15 minutes à froid, lavé à l'acétone et placé rapidement dans un dessicateur sous vide. On obtient ainsi 620 mg (72 %) de microcristaux de dichlorhydrate de [(diméthylamino-3) propyl]-amino-1 hydroxy-9 méthyl-4-5H-pyrido [4,3-b] benzo [e] indole hydraté (1,5 $H_2O$), F = 250-260 °C avec décomposition.

Le [(diméthylamino-3)propyl]amino-1 hydroxy-9 méthyl-4-5H-pyrido [4,3-b] benzo [e] indole (350 mg) en solution dans l'acétone à l'ébullition (100 $cm^3$) est versé dans une solution d'acide maléique (350 mg, 3 équivalents) dans l'acétone bouillante (30 $cm^3$) et le mélange est laissé en flacon bouché pendant une nuit à la température ambiante. Le solide obtenu est filtré, lavé à l'acétone et placé pendant 18 heures sous vide pour donner des microcristaux beiges de di-maléate de [(diméthylamino-3)propyl] amino-1 hydroxy-9 méthyl-4-5H-pyrido [4,3-b] benzo [e] indole, F = 204-206 °C.

Le [(diméthylamino-3)propyl]amino-1 méthoxy-9 méthyl-4-5H-pyrido [4,3-b] benzo [e] indole peut être préparé de la manière suivante :

Le chloro-1 méthoxy-9 méthyl-4-5H-pyrido [4,3-b] benzo [e] indole (7,2 g, 24 mmol) est placé dans un ballon de 500 $cm^3$ contenant 120 $cm^3$ (large excès) de diméthylamino-3 propylamine et chauffé à reflux, sous azote et sous agitation, pendant 72 heures, (disparition totale du dérivé chloré, contrôlé sur plaque de silice).

L'excès de la diamine est évaporé au bain-marie sous pression réduite et le résidu est repris dans l'eau puis alcalinisé par l'ammoniaque. Le solide formé est filtré, lavé à l'eau, repris dans le chlorure de méthylène et lavé avec 150 $cm^3$ d'eau additionnée de 10 $cm^3$ d'ammoniaque.

La solution organique est séchée, filtrée et évaporée pour donner un résidu solide correspondant au [-(diméthylamino-3) propyl]amino-1 méthoxy-9 méthyl-4-5H-pyrido[4,3-b] benzo [e] indole pur en CCM (8,7 g).

Le chloro-1 méthoxy-9 méthyl-4-5H-pyrido[4,3-b] benzo [e] indole peut être obtenu de la manière suivante :

Dans un ballon de 1 litre, muni d'un agitateur magnétique, d'une ampoule à addition, d'un réfrigérant et

maintenu sous azote, on introduit 12,2 g (43 mmol) de méthoxy-9 méthyl-4-2H, 5H-pyrido [4,3-b] benzo [e] indolone-1, 10,4 g (172 mmol) d'acétamide, 40 g (172 mmol) de chlorure de benzyl triéthylammonium, 110 cm$^3$ d'acétonitrile et 28 cm$^3$ (172 mmol) de diéthylaniline fraichement rectifiée.

Par l'ampoule, on ajoute progressivement 200 cm$^3$ d'oxychlorure de phosphore rectifié, et observe une réaction exothermique. Le mélange est chauffé à reflux pendant 10 heures, tandis qu'après être passé par une phase homogène, un précipité apparaît. Après le temps indiqué, on concentre à sec, au bain marie chauffé à 70° C et sous pression réduite (15 mm de mercure).

Au résidu obtenu, on ajoute 300 cm$^3$ d'eau glacée et le mélange, bien agité pendant 2 minutes, est chauffé à l'ébullition. Il se forme un précipité jaune, que l'on filtre à froid et lave ensuite à l'eau froide. Ce précipité est repris dans 300 cm$^3$ d'eau distillée et le mélange est alcalinisé, à froid, par l'ammoniaque. L'ensemble est laissé sous agitation pendant 1 heure et le précipité est filtré et lavé à l'eau. Le contrôle en chromatographie sur couche mince montre la présence du composé attendu (rf = 0,5) et la présence de traces du composé de départ (rf = 0,36) ($SiO_2$, $CH_2Cl_2$-EtOH, 9/l). Le produit attendu cristallise dans l'alcool dans lequel il est très peu soluble (gros volumes) en donnant 12 g (92 %) d'aiguilles jaunes, F > 270° C (rf = 0,58 sur plaque silice, éluant acétate d'éthyle pur).

Le méthoxy-9 méthyl-4-2H,5H-pyrido [4,3-b] benzo [e] indolone-1 peut être préparé de la manière suivante :

Dans un tricol de 4 litres, on mélange la N-(méthyl-5-1H -pyridone-2 yl)-4 N'-(méthoxy-6 tétrahydro-1,2,3,4-naphtalènylidène)-2 hydrazine (31 g, 0,1 mol) et le diphényléther (1 litre) puis chauffe à reflux pendant 40 minutes, en maintenant l'ensemble sous bonne agitation et sous azote. Le mélange devient homogène et se décolore. Le chauffage est interrompu pour laisser refroidir à 200° C et contrôler la transformation du composé de départ. En continuant à agiter, le charbon palladié à 10 % (5 g) en suspension dans le diphényléther (100 cm$^3$) est ajouté progressivement, avec précautions (mousses et dégagement d'hydrogène), et le nouveau mélange est de nouveau chauffé à reflux pendant 40 minutes. L'hexane (2 litres) est ajouté au mélange refroidi et le précipité formé est filtré puis lavé à l'hexane. Il est alors repris dans 1 litre d'acide acétique bouillant, filtré pour éliminer le charbon palladié et ce dernier est lavé une fois avec 50 à 100 cm$^3$ d'acide acétique bouillant. L'ensemble du filtrat est concentré à 500 cm$^3$ et le solide obtenu après refroidissement est filtré, lavé à l'acétone bouillante et séché pour donner 22 g (75 %) de microcristaux jaune pâle, F = 260° C, correspondant à la méthoxy-9 méthyl-4-2H,5H-pyrido[4,3-b] benzo[e]indolone-1 attendue, légèrement hydratée (rf 0,36 sur plaque de silice, avec le mélange $CH_2Cl_2$-EtOH 9/l comme éluant).

La N-(méthyl-5-1H-pyridone-2 yl)-4 N'-(méthoxy-6 tétrahydro-1,2,3,4-naphtalènylidène)-2 hydrazine peut être préparée de la manière suivante :

Le mélange de l'hydrazino-4 méthyl-5-1H-pyridone-2 (13,9 g, 0,1 mol) dans l'éthanol absolu (600 cm$^3$) est chauffé à reflux. A la solution, devenue homogène à l'ébullition, on ajoute la méthoxy-6 tétralone-2 (11,3 g, 0,12 mol) et le mélange, devenu rapidement hétérogène, est chauffé à reflux pendant 4 heures 30 minutes. Le précipité formé est filtré, repris dans 400 cm$^3$ d'étha nol bouillant dans lequel l'hydrazone est très peu soluble puis filtré à froid pour donner 15,7 g (98 %) de microcristaux incolores, F = 240-250° C, avec décomposition.

EXEMPLE 2

Le dimaléate de (diméthylamino-3 méthyl-2 propyl) amino-1 méthoxy-9 méthyl-4-5H-pyrido[4,3-b] benzo [e] indole-(R,S) (3 g) est dissous dans l'eau et additionné d'ammoniaque. Après extraction par 3 fois 100 cm$^3$ de chlorure de méthylène séchage et concentration à sec, on obtient la base libre correspondante qui est utilisée telle quelle dans la suite de la synthèse.

Le (diméthylamino-3 méthyl-2 propyl) amino-1 méthoxy-9 méthyl-4-5H-pyrido[4,3-b] benzo [e] indole-(R,S) est repris dans 120 cm$^3$ d'acide bromhydrique (d = 1,47, 47 %) en opérant de manière analogue à l'exemple 1.

Après chauffage à reflux pendant 4 heures, et refroidissement, un précipité apparaît. Le mélange réactionnel est concentré à sec sous pression réduite, puis le résidu est additionné de 200 cm$^3$ d'eau et la solution obtenue est filtrée pour éliminer un léger insoluble. Le filtrat est additionné de 20 cm$^3$ de triéthylamine et de 30 cm$^3$ d'ammoniaque concentré, puis de 200 cm$^3$ de chlorure de méthylène. Après agitation pendant 1 heure à température ambiante, le mélange est extrait par 4 fois 100 cm$^3$ de chlorure de méthylène, séché et concentré à sec.

Le résidu solide est chromatographié sur alumine en éluant successivement par 500 cm$^3$ de chlorure de méthylène, 1 litre d'un mélange (97-3) de chlorure de méthylène et d'éthanol, et 1 litre d'un mélange

(80-20) de chlorure de méthylène et d'éthanol. Les fractions correspondant au dernier litre d'éluant sont recueillies et concentrées à sec. On obtient ainsi 2,1 g de (diméthylamino-3 méthyl-2 propyl)amino-1 hydroxy-9 méthyl-4 5H-pyrido[4,3-b] benzo[e] indole-(R,S) brut.

Le produit obtenu est dissous dans 200 $cm^3$ d'acétone et porté à ébullition, puis additionné d'acide maléique (2,1 g, 3 équivalents) en solution dans 50 $cm^3$ d'acétone bouillante. Le mélange est concentré à moitié de son volume et laissé refroidir. On obtient ainsi 2,25 g de dimaléate de (diméthylamino-3 méthyl-2 propyl)amino-1 hydroxy-9 méthyl-4 5H-pyrido [4,3-b] benzo [e] indole-(R,S) fondant à 215-220 °C.

Le dimaléate de (diméthylamino-3 méthyl-2 propyl) amino-1 méthoxy-9 méthyl-4-5H-pyrido [4,3-b] benzo [e] indole-(R,S) peut être obtenu de la manière suivante :

Le chloro-1 méthoxy-9 méthyl-4-5H-pyrido [4,3-b] benzo [e] indole (2,1 g) est chauffé en autoclave à 180 °C pendant 16 heures avec la diméthylamino-3 méthyl-2 propylamine.

On opère dans les conditions décrites à l'exemple 1, alcalinise par l'ammoniaque reprend par le chlorure de méthylène et chromatographie sur alumine en éluant par 1,5 litre de chlorure de méthylène. Après concentration à sec, le résidu obtenu est dissous dans 60 $cm^3$ d'acétone et chauffé 2 minutes avec de l'acide maléique (2,5 g, 3 équivalents) dissous dans 50 $cm^3$ d'acétone. Après refroidissement et précipitation on obtient 3,2 g de dimaléate de (diméthylamino-3 méthyl-2 propyl) amino-1 méthoxy-9 méthyl-4-5H- pyrido [4,3-b] benzo [e] indole-(R,S) fondant à 180 °C.

EXEMPLE 3

En opérant de manière analogue à l'exemple 2 mais à partir de tri-méthanesulfonate de [-(diméthylamino-3)propyl]amino-1 méthoxy-9 diméthyl-4,5 pyrido [4,3-b] benzo [e] indole hydraté ($2H_2O$), on obtient le di-méthanesulfonate de [(diméthylamino-3)propyl] amino-1 hydroxy-9 diméthyl-4,5 pyrido [4,3-b] benzo [e] indole hydraté (1,5 $H_2O$), F = 135-150 °C avec un rendement de 95 %.

Le tri-méthanesulfonate de [(diméthylamino-3)propyl] amino-1 méthoxy-9 diméthyl-4,5 pyrido [4,3-b] benzo [e] indole hydraté ($2H_2O$) peut-être obtenu comme décrit à l'exemple 2 mais à partir du chloro-1 méthoxy-9 diméthyl-4,5 pyrido [4,3-b] benzo [e] indole. Le produit est obtenu avec un rendement de 86 %, F = 115-125 °C.

Le chloro-1 méthoxy-9 diméthyl-4,5 pyrido [4,3-b] benzo [e] indole peut-être obtenu de la manière suivante :

Le chloro-1 méthoxy-9 méthyl-4 5H pyrido [4,3-b] benzo [e] indole (15 mmol) est dissous dans 100 $cm^3$ de diméthylformamide et traité sous agitation, à température ambiante, pendant 15 heures, par 1,15 $cm^3$ (18 mmol) d'iodure de méthyle en présence de 16,6 g (120 mmol) de carbonate de potassium. Le mélange réactionnel est concentré à sec sous pression réduite.

Le résidu est repris dans l'eau et extrait par du chlorure de méthylène. La solution est séchée sur sulfate de magnésium et concentrée à sec. Le résidu solide est recristallisé dans l'éthanol pour donner le chloro-1 méthoxy-9 diméthyl-4,5 pyrido [4,3-b] benzo [e] indole sous forme d'aiguilles blanches avec un rendement de 86 % ; F = 185-187 °C.

EXEMPLE 4

Le [(diméthylamino-2)éthyl amino-1 méthoxy-9 méthyl-4-5H-pyrido [4,3-b] benzo [e] indole est placé dans un ballon réactionnel de 250 $cm^3$ muni d'un agitateur et maintenu sous argon, avec 80 $cm^3$ d'acide bromhydrique (d = 1,47,47%).

Ce mélange, devenu homogène à l'ébulition, est chauffé à reflux pendant 5 heures et concentré à sec sous pression réduite. Le résidu solide est dissous dans l'eau (400 $cm^3$) alcalinisé par l'ammoniaque à 28% (18 $cm^3$) que l'on ajoute goutte à goutte, pour former un précipité gommeux.

Au mélange résultant, on ajoute 200 $cm^3$ d'acétate d'éthyle, agite l'ensemble pendant une nuit et filtre. La phase organique est décantée et les eaux mères sont extraites deux fois avec 100 $cm^3$ d'acétate d'éthyle. La phase organique est séchée sur sulfate de sodium, filtrée, traitée avec 4 g de charbon animal pour la décolorer, filtrée à nouveau et concentrée à sec.

Le résidu solide est repris dans 30 $cm^3$ d'acétone et 50 $cm^3$ d'éthanol absolu, et le mélange homogène est filtré à chaud dans une solution d'acide maléique (3 équivalents) dans 30 $cm^3$ d'éthanol absolu pour donner, après évaporation du solvant et reprise du résidu dans l'acétone, le dimaléate du [(diméthylamino-2) éthyl] amino-1 hydroxy-9 méthyl-4-5H-pyrido [4,3-b] benzo [e] indole, F = 210 °C. Le rendement pour les trois étapes: substitution, déméthylation et salification s'élève à 76%.

Le [(diméthylamino-2)éthyl]amino-1 méthoxy-9 méthyl-4-5H-pyrido [4,3-b] benzo [e] indole peut être préparé de la manière suivante;

Le chloro-1 méthoxy-9 méthyl-4-5H-pyrido [4,3-b] benzo [e] indole (2 g) est placé dans un ballon de 50 $cm^3$ contenant 20 $cm^3$ (large excès) de diméthylamino-2-éthylamine et chauffé à reflux (120 °C) sous azote et sous agitation, pendant 16 jours (disparition totale du dérivé chloré, contrôlée sur plaque de silice).

L'excès de la diamine est évaporé au bain-marie sous pression réduite et le résidu est repris dans l'eau puis alcalinisé par l'ammoniaque. Le soldie formé est filtré, lavé à l'eau, repris dans le chlorure de méthylène et lavé avec 150 $cm^3$ d'eau et 10 $cm^3$ d'ammoniaque.

La solution organique est séchée, filtrée et concentrée à sec pour donner un résidu solide correspondant au maléate du [(diméthylamino-2)éthyl] amino-1 méthoxy-9 méthyl-4-5H-pyrido [4,3-b] benzo [e] indole; F = 184 °C.

## EXEMPLE 5

L' [(amino-3) propyl] amino-1 méthoxy-9 méthyl-4-5H-pyrido [4,3-b] benzo [e] indole (1,9 g) est placé dans un ballon réactionnel de 250 $cm^3$ muni d'un agitateur et maintenu sous argon, avec 70 cm3 d'acide bromhydrique (d = 1,47; 47%).

Ce mélange, devenu homogène à l'ébullition, est chauffé à reflux pendant 5 heures 30 minutes et concentré à sec sous pression réduite. Le résidu solide est dissous dans l'eau (200 $cm^3$) alcalinisé par l'ammoniaque à 28% (20 $cm^3$) que l'on ajoute goutte à goutte, pour former un précipité gommeux.

Au mélange résultant, on ajoute 200 $cm^3$ d'acétate d'éthyle et 50 $cm^3$ d'éthanol, agite l'ensemble pendant 1 heure et filtre pour éliminer une petite fraction insoluble. La phase organique est décantée et les eaux mères sont extraites deux fois avec 100 $cm^3$ d'acétate d'éthyle. La phase organique est séchée sur sulfate de sodium, filtrée, traitée avec 4 g de charbon animal pour la décolorer, puis filtrée et concentrée à sec.

Le résidu solide est repris dans 30 $cm^3$ d'acétone, agité pendant 15 mn et filtré puis lavé 2 fois avec 10 $cm^3$ d'acétone froide pour donner 1,5 g (80%) d'un solide correspondant à l'[(amino-3) propyl] amino-1 hydroxy-9-méthyl-4-5H-pyrido [4,3-b] benzo [e] indole, hydraté avec 1,3 molécules d'eau dont le bimaléate, recristallisé dans le mélange éthanol-acétone, fond à 132-136 °C.

L'[(amino-3) propyl] amino-1 méthoxy-9 méthyl-4-5H-pyrido [4,3-b] benzo [e] indole peut être préparé de la manière suivante:

Le dérivé chloré (2,5 g) est placé dans un ballon de 250 $cm^3$ (large excès) d'amino-3 propylamine et chauffé à 160 °C sous azote et sous agitation pendant 18 heures (disparition totale du dérivé chloré, contrôlé sur plaque de silice).

L'excès de la diamine est évaporé au bain-marrie sous pression réduite et le résidu est repris dans l'eau puis alcalinisé par l'ammoniaque. Le solide formé est filtré, lavé à l'eau, et séché au dessicateur pour donner 2,8 g d'un produit solide correspondant au composé attendu.

Le bimaléate, préparé dans l'éthanol absolu en présence d'un excès d'acide maléique et lavé à l'acétone, correspond au sel de l'[amino-3) propyl] amino-1 méthoxy-9 méthyl-4-5H-pyrido [4,3-b] benzo [e] indole, monohydraté; F = 170 °C.

## EXEMPLE 6

Le [(diméthylamino-3) propyl] amino-1 éthyl-4 méthoxy-9 5H-pyrido [4,3-b] benzo [e] indole (base libre) est placé dans un ballon réactionnel de 250 $cm^3$ muni d'un agitateur sous argon, avec 66 $cm^3$ d'acide bromhydrique (d = 1,47; 47%).

Ce mélange, devenu homogène à l'ébullition, est chauffé à reflux pendant 5 heures et concentré à sec sous pression réduite. Le résidu solide est dissous dans l'eau (200 $cm^3$) alcalinisé par l'ammoniaque à 28% (15 $cm^3$) que l'on ajoute goutte à goutte, pour former un précipité gommeux.

Au mélange résultant, on ajoute 200 $cm^3$ d'acétate d'éthyle et agite l'ensemble pendant une heure. La phase organique est décantée et les eaux mères sont extraites deux fois avec 100 $cm^3$ d'acétate d'éthyle. La phase organique est séchée sur sulfate de sodium, filtrée, traitée avec 5 g de charbon animal pour la décolorer, puis filtrée et concentrée à sec.

Le résidu solide est repris dans 40 $cm^3$ d'acétone, et traité avec 2,5 g d'acide maléique en solution dans 40 $cm^3$ d'acétone à l'ébullition. Le bimaléate du [(diméthylamino-3) propyl] amino-1 éthyl-4 hydroxy-9 5H-pyrido [4,3-b] benzo [e] indole (2,34 g, Rdt = 72% pour les 3 étapes: substition - déméthylation -

salification) est filtré et séché pour donner des microcristaux crème; F = 154° C. Le [(diméthylamino-3) propyl] amino-1 éthyl-4 méthoxy-9 5H-pyrido [4,3-b] benzo [e] indole peut être obtenu de la manière suivante.

Le chloro-1 éthyl-4 méthoxy-9 5H-pyrido [4,3-b] benzo [e] indole (2 g) est placé dans un ballon de 250 $cm^3$ contenant 40 $cm^3$ (large excès) de diméthylamino-3 propylamine et chauffé à reflux, sous azote et sous agitation, pendant 72 heures (disparition totale du dérivé chloré, contrôlé sur plaque de silice).

L'excès de la diamine est évaporé au bain-marie sous pression réduite et le résidu est repris dans l'eau puis alcalinisé par l'ammoniaque. Le solide formé est filtré, lavé à l'eau, repris dans le chlorure de méthylène et lavé avec 150 $cm^3$ d'eau et 10 $cm^3$ d'ammoniaque.

La solution organique est séchée, filtrée et évaporée pour donner un résidu solide correspondant au [-(diméthylamino-3) propyl]amino-1 éthyl-4 méthoxy-9 5H-pyrido [4,3-b] benzo [e] indole attendu.

Le bimaléate correspondant, formé dans les conditions usuelles fond à 154° C.

Le chloro-1 éthyl-4 méthoxy-9 5H-pyrido [4,3-b] benzo [e] indole peut être obtenu de la manière suivante:

Dans un ballon de 500 $cm^3$, muni d'un agitateur magnétique, d'une ampoule à addition, d'un réfrigérant et maintenu sous azote, on introduit 6 g de méthoxy-9 éthyl-4-2H, 5H-pyrido [4,3-b] benzo [e] indolone-1 dans l'éthanol bouillant et séché, 19,2 g de chlorure de benzyl triéthylammonium, 5,1 g d'acétamide, 60 $cm^3$ d'acétonitrile et 13,2 g de diéthylaniline fraichement rectifiée.

Par l'ampoule, on ajoute progressivement 96 $cm^3$ d'oxy chlorure de phosphore rectifié, et observe une réaction exothermique. Le mélange est chauffé à reflux pendant 6 heures 30 minutes, tandis qu'après être passé par une phase homogène, un précipité apparaît. On évapore à sec, au bain marie chaufé à 70° C et sous vide de 15 mm.

Au résidu obtenu, on ajoute 600 $cm^3$ d'eau glacée et le mélange, bien agité pendant 2 heures, est chauffé à l'ébullition. Après avoir refroidi, on obtient un précipté jaune, que l'on filtre à froid et lave ensuite à l'eau froide. Ce précipité est repris dans 500 $cm^3$ d'eau distillée et le mélange est alcalinisé, à froid, par l'ammoniaque. L'ensemble est laissé sous agitation pendant 1 heure 30 minutes et le précipité est filtré et lavé à l'eau. Il recristallise dans l'alcool en donnant 4,6 g d'aiguilles jaunes de chloro-1 éthyl-4 méthoxy-9 5H-pyrido [4,3-b] benzo [e] indole; F = 260° C (Rendement 62%).

La méthoxy-9 éthyl-4-2H,5H-pyrido [4,3-b] benzo [e] indolone-1 peut être obtenue de la manière suivante:

Dans un tricol de 1 litre, on mélange la N-éthyl-5-1H-pyridone-2 yl)-4 N′-méthoxy tétrahydro-1,2,3,4-naphtalénylidène)-2 hydrazine (14,5 g) et le diphényléther (350 $cm^3$ puis chauffe à reflux pendant 30 minutes, en maintenant l'ensemble sous bonne agitation et sous azote. Le mélange devient homogène et se décolore. Le chauffage est interrompu pour laisser refroidir à 200 °C et contrôler que le composé de départ est entièrement transformé; plaque de silice Merk, éluant chlorure de méthylène-éthanol 8/2 en volumes, (rf hydrazone = 0,5, rf composé intermédiaire = 0,8). En continuant à agiter, le charbon palladié à 10% (2g) en suspension dans le diphényléther (20 $cm^3$) est ajouté progressivement, avec précaution (mousses et dégagement d'hydrogène), et le nouveau mélange est chauffé à reflux pendant 30 minutes (disparition du composé intermédiaire) (rf = 0,35 silice-acétate d'éthyle pur; produit attendu; rf = 0,58). L'hexane (400 $cm^3$) est ajouté au mélange refroidi et le précipité formé est filtré puis lavé à l'hexane. Il est alors repris dans 350 $cm^3$ d'acide acétique bouillant, filtré pour éliminer le charbon palladié et ce dernier lavé deux fois avec 30 $cm^3$ d'acide acétique bouillant.

L'ensemble du filtrat est concentré à 150 $cm^3$ et le solide obtenu après refroidissement est filtré et séché sous vide pour donner 13,7 g (Rdt quantatif) de microcristaux jaune pâle (F > 260° C), correspondant à la méthoxy-9 éthyl-4-2H,5H-pyrido [4,3-b] benzo [e] indolone-1 hydratée.

La N-éthyl-5-1H-pyridone-2 yl)-4 N′-méthoxy tétrahydro-1,2,3,4- naphtalènylidène)-2 hydrazine peut être obtenue de la manière suivante:

Le mélange de l'hydrazino-4 éthyl-5-1H-pyridone-2 (7 g) dans l'éthanol absolu (250 $cm^3$) est chauffé à reflux. A la solution, devenue homogène à l'ébullition, on ajoute la méthoxy-6 tétralone-2 (9/3 g) et le mélange, devenu rapidement hétérogène, est chauffé à reflux pendant 4 heures 30 minutes (disparition de l'hydrazine contrôlée par CCM sur plaque de silice Merk, éluant chlorure de méthylène-éthanol 7/3 en volumes, rf = 0,75). Le précipité formé est filtré, repris dans 100 $cm^3$ d'éthanol dans lequel l'hydrazone est peu soluble puis filtré à froid pour donner (12,1 g (85%)) de microcristaux incolores de N-éthyl-5-1H-pyridone-2 yl)-4 N′-méthoxytétrahydro-1,2,3,4- naphtalènylidène)-2 hydrazine, F = 135 - 140° C, avec décomposition.

L'hydrazino-4 éthyl-5-1H-pyridone-2 peut être obtenue de la manière suivante:

Dans un flacon réactionnel placé sous azote et muni d'un agitateur magnétique ainsi que d'un réfrigérant, on mélange 12 g de l'hydroxy-4 éthyl-5-1H-pyridone-2, 40 $cm^3$ d'éther monoéthylique de

l'éthylène glycol et 11 $cm^3$ d'hydrate d'hydrazine.

L'ensemble est chauffé à reflux, sous agitation, pendant 4 jours au total.

Après avoir laissé reposer à froid pendant une nuit, le solide obtenu est filtré, lavé avec 10 $cm^3$ d'éthanol absolu froid et séché pour donner 7,8 g (59%) de l'hydrazino-4 éthyl-5-1H-pyridone-2 monohydratée, F = 130-132 °C, avec décomposition.

L'hydroxy-4 éthyl-5-1H-pyridone-2 est décrite par M. Legraverend, C.H. Nguyen, A. Zérial et E. Bisagni Nucléosides and Necléotides 5 125-134 (1986).

La présente invention concerne également les compositions pharmaceutiques qui contiennent comme produit actif au moins un produit de formule générale (I) à l'état pur (sous forme libre ou sous forme de sel) ou en association avec un ou plusieurs adjuvants pharmaceutiquement acceptables. Ces compositions peuvent être utilisées par voie parentérale.

Les compositions pour administration parentérale, peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui seront dissoutes au moment de l'emploi dans de l'eau stérile ou tout autre milieu stérile injectable.

En thérapeutique humaine, les médicaments selon la présente invention sont particulièrement utiles dans le traitement des cancers digestifs, des cancers pulmonaires, des cancers des testicules ou des ovaires ainsi que dans les traitements des cancers de la tête et du cou.

D'une façon générale, le médecin déterminera la posologie qu'il estime la plus appropriée en fonction de l'âge, du poids et de tous les autres facteurs propres au sujet à traiter.

Le mode d'administration préféré est la voie intraveineuse. A titre indicatif, les médicaments selon l'invention peuvent être administrés chez l'homme à raison de 30 à 200 $mg/m^2$ par traitement, par voie intra-veineuse.

L'exemple suivant, donné à titre non limitatif, illustre une composition selon la présente invention :

EXEMPLE :

On prépare une solution contenant 6,44 g de dichlorhydrate de [(diméthylamino-3)propyl] amino-1 hydroxy-9 méthyl-4 5H-pyrido [4,3-b] benzo [e] indole hydraté (1,5 $H_2O$), en dissolvant ce produit dans du soluté physiologique apyrogène en quantité suffisante pour obtenir 100 $cm^3$.

La solution obtenue est répartie aseptiquement en ampoules, à raison de 2 $cm^3$ par ampoule. Les ampoules sont scellées et contiennent chacune 100 mg de [(diméthylamino-3)propyl] amino-1 hydroxy-9 méthyl-4 5H-pyrido [4,3-b] benzo [e] indole.

## Revendications

1 - Un nouveau dérivé de pyridobenzoindole de formule générale :

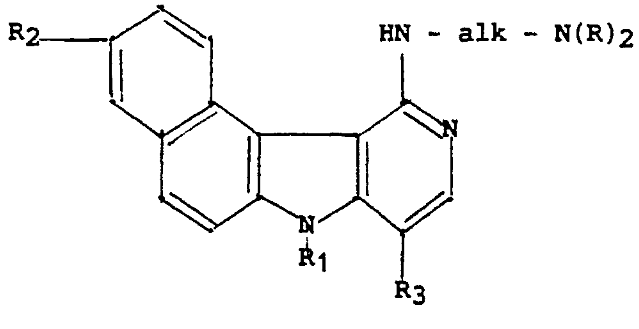

dans laquelle

R représente un atome d'hydrogène ou un radical alcoyle contenant 1 ou 2 atomes de carbone,
alk représente un radical alcoylène droit ou ramifié contenant 2 à 4 atomes de carbone,
$R_1$ représente un atome d'hydrogène ou un radical alcoyle contenant 1 ou 2 atomes de carbone,
$R_2$ représente un radical hydroxy ou méthoxy, et
$R_3$ représente un radical alcoyle contenant 1 ou 2 atomes de carbone, ainsi que ses sels d'addition avec les acides, et le cas échéant ses hydrates et ses formes isomères et leurs mélanges.

2 - Un nouveau dérivé du pyridobenzoindole selon la revendication 1 pour lequel:
R est un atome d'hydrogène ou un radical méthyle,
alk est un radical alcoylène droit ou ramifié contenant 2 à 4 atomes de carbone,
$R_1$ est un atome d'hydrogène ou un radical méthyle,
$R_2$ est radical hydroxy et
$R_3$ est un radical méthyle ou éthyle,
ainsi que ses sels et le cas échéant ses hydrates et ses formes isomères et leurs mélanges.

3 - Un nouveau dérivé du pyridobenzoindole selon la revendication 1 pour lequel:
R est un radical méthyle,
alk est un radical alcoylène droit ou ramifié contenant 2 à 4 atomes de carbone,
$R_1$ est un atome d'hydrogène ou un radical méthyle,
$R_2$ est un radical hydroxy, et
$R_3$ est un radical méthyle, ainsi que ses sels et le cas échéant ses hydrates et ses formes isomères et leurs mélanges.

4 - Le [(diméthylamino-3) propyl] amino-1 hydroxy-9 méthyl-4-5H-pyrido [4,3-b] benzo [e] indole, ainsi que ses sels et ses formes hydratées.

5 - Le (diméthylamino-3 méthyl-2 propyl) amino-1 hydroxy-9 méthyl-4-5H-pyrido[4,3-b] benzo [e] indole, ainsi que ses sels, ses formes hydratées et ses formes isomères et leurs mélanges.

6 - Un procédé de préparation d'un dérivé de pyridobenzoindole selon la revendication 1, caractérisé en ce que l'on fait agir une amine de formule générale :
$H_2N$ - alk - $N(R)_2$
dans laquelle alk et R sont définis comme dans la revendication 1, sur un dérivé chloré de formule :

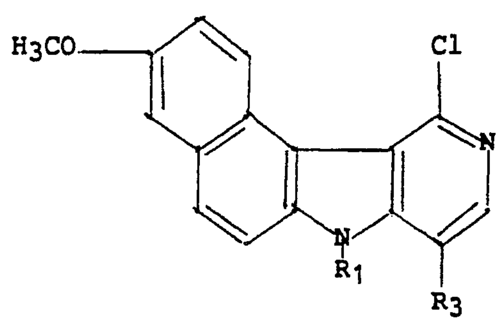

dans laquelle $R_1$ et $R_3$ sont définis comme dans la revendication 1, puis transforme le cas échéant le produit méthoxylé obtenu, de formule générale:

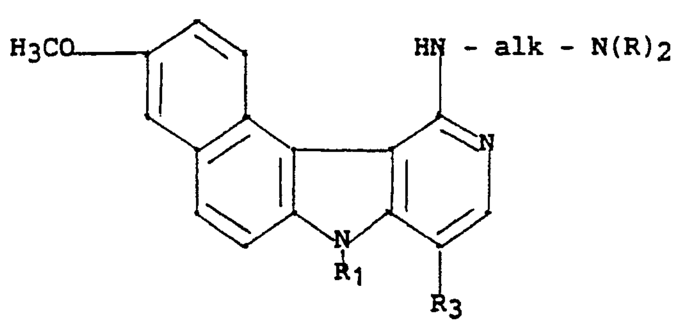

dans laquelle $R_1$ et $R_3$ sont définis comme dans la revendication 1, en un dérivé hydroxy-9 pyrido [4,3-b] benzo [e] indole et transforme éventuellement le produit obtenu en un sel d'addition avec un acide.

7 - Composition pharmaceutique caractérisée en ce qu'elle contient un produit selon la revendication 1

à l'état pur ou sous forme d'association avec tout diluant ou adjuvant compatible et pharmaceutiquement acceptable.

Revendications pour les Etats contractants suivants: ES, GR.

Procédé de préparation d'un nouveau dérivé de pyridobenzoindole de formule générale :

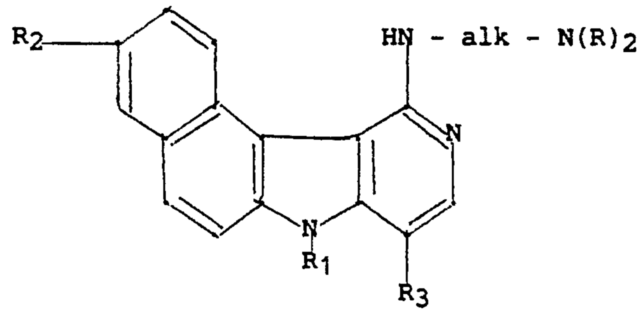

dans laquelle
R représente un atome d'hydrogène ou un radical alcoyle contenant 1 ou 2 atomes de carbone,
alk représente un radical alcoylène droit ou ramifié contenant 2 à 4 atomes de carbone,
$R_1$ représente un atome d'hydrogène ou un radical alcoyle contenant 1 ou 2 atomes de carbone,
$R_2$ représente un radical hydroxy ou méthoxy, et
$R_3$ représente un radical alcoyle contenant 1 ou 2 atomes de carbone, ainsi que ses sels d'addition avec lés acides, et le cas échéant ses hydrates et ses formes isomères et leurs mélanges, caractérisé en ce que l'on fait agir une amine de formule générale:
$H_2N$ - alk - $N(R)_2$
dans laquelle alk et R sont définis comme dans la revendication 1, sur un dérivé chloré de formule :

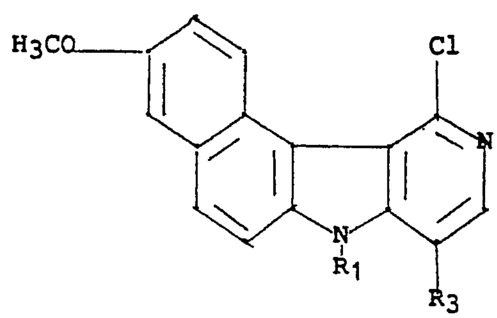

dans laquelle $R_1$ et $R_3$ sont définis comme ci-dessus, puis transforme le cas échéant le produit méthoxylé obtenu, de formule générale:

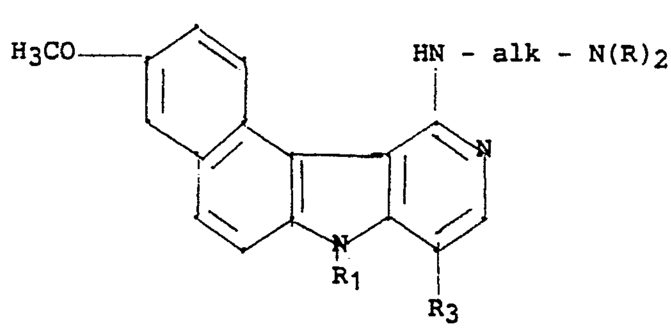

dans laquelle $R_1$ et $R_3$ sont définis comme ci-dessus, en un dérivé hydroxy-9 pyrido [4,3-b] benzo [e] indole et transforme éventuellement le produit obtenu en un sel d'addition avec un acide.

Office européen des brevets

# RAPPORT DE RECHERCHE EUROPEENNE

Numero de la demande

EP 90 40 1512

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 226 508 (SANOFI)<br>* Revendications 1,9; page 10, lignes 10-37; pages 11-19; page 20, lignes 1-32 *<br>--- | 1,7 | C 07 D 471/04<br>A 61 K 31/435 //<br>(C 07 D 471/04<br>C 07 D 221:00<br>C 07 D 209:00 ) |
| D,A | EP-A-0 239 476 (SANOFI)<br>* Revendications 1,11; pages 19-37 *<br>----- | 1,7 | |
| | | | **DOMAINES TECHNIQUES RECHERCHES (Int. Cl.5)** |
| | | | C 07 D 471/00<br>A 61 K 31/00 |

Le présent rapport a été établi pour toutes les revendications

| Lieu de la recherche | Date d'achèvement de la recherche | Examinateur |
|---|---|---|
| LA HAYE | 10-09-1990 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)